# EUROPEAN PATENT SPECIFICATION

(11) **EP 3 045 179 B1**
(45) Date of publication and mention of the grant of the patent: **01.05.2019**
(21) Application number: 14838087.6
(22) Date of filing: 10.07.2014
(51) Int. Cl.: A61K 33/00, A61P 23/02, A61K 31/573, A61K 31/5415, A61K 31/51, A61K 31/714, A61K 31/4415, A61K 31/525

(54) **LOCAL ANESTHESIA PAIN-RELIEVING TIME-DELAY AGENT**
MITTEL ZUR ZEITVERZÖGERUNG DER LOKALANÄSTHESIESCHMERZLINDERUNG
AGENT DE TEMPORISATION D'ATTÉNUATION DE LA DOULEUR À ANESTHÉSIE LOCALE

(30) Priority: 20.08.2013 CN 201310364295
(43) Date of publication of application: 20.07.2016
(73) Proprietor: Li, Fuchao, Chaoyang District Beijing 100013 (CN)
(72) Inventor: Li, Fuchao, Chaoyang District Beijing 100013 (CN)
(74) Representative: Hoppe, Georg Johannes
(86) International application number: PCT/CN2014/081969
(87) International publication number: WO 2015/024420

(56) References cited:
- CN-A- 1 266 685
- CN-A- 1 709 504
- CN-A- 103 239 456
- CN-A- 103 393 714
- US-B2- 7 928 141
- CARAM-SALAS NADIA L ET AL: "Antinociceptive synergy between dexamethasone and the B vitamin complex in a neuropathic pain model in the rat", WESTERN PHARMACOLOGY SOCIETY. PROCEEDINGS, WESTERN PHARMACOLOGY SOCIETY, TUCSON, AZ, US, vol. 47, 1 January 2004 (2004-01-01), pages 88-91, XP009102472, ISSN: 0083-8969
- T H Braid ET AL: "E", , 1 January 1966 (1966-01-01), XP055329865, Retrieved from the Internet: URL:http://onlinelibrary.wiley.com/store/1 0.1111/j.1399-6576.1966.tb01031.x/asset/j. 1399-6576.1966.tb01031.x.pdf?v=1&t=iwrqmhy 8&s=d5471ea4d01b63473edc13cd2427aea2bbe2cb 8a [retrieved on 2016-12-16]

## Description

### Technical Field

The present invention relates to the field of anethesia for surgery of human beings and animals, and specifically to an analgesic composition for prolonging the duration of action of a local anesthetic.

### Background Art

Currently, anesthesia methods used in a variety of operations include two kinds of general anesthesia and local anesthesia. Hereinafter, the term "general anesthesia" may be abbreviated as the term "GA" and the term "local anesthesia" may be abbreviated as the term "LA."

General anesthesia refers to an anesthesia method of inhibiting central nerve of the brain, thereby causing a patient to temporally lose one's consciousness to achieve the purpose of pain relieving. However, in case a general anesthesia method is used, an anesthetic specialist and special anesthetic equipment are required, a surgical risk is relatively high, nursing of a patient after surgery is complicated, and a patient cannot move by oneself.

Local anesthesia referres to an anesthesia method of reversely blocking transmission of sensory nerve by locally using an anesthetic, thus causing a patient to temporarily lose pain of local tissues while having a clear consciousness. In case a local anesthesia method is used, it has little influence on the physiological functions of the whole body, side effects are little, and a patient has a clear consciousness during surgery, thus making observation easy, which is advantageous for a doctor to grasp the condition of the patient. And, since a patient can move by oneself after surgery, manpower and material power may be reduced, and it is often used for emergency rescue and patient transfer when the emergency occurs.

China has a large territory and abundant natural resources, but a variety of natural disasters frequently ocurr. For example, earthquake occurred in the areas of China Wenchuan, Yushu, Ya an and the like may be mentioned. After earthquake occurred in the areas of China Wenchuan, Yushu, Ya an and the like, lack of analgesic injections, anesthetics, anesthetic specialists, anesthetic equipment and the like were experienced during on-site lifesaving operations. It can be said that such a phenomenon clearly shows the present state of China with serious lack of specialists and drugs. Currently, it is urgently needed to develop an analgesic that is easy to use and can continue local pain relieving anesthetic effect for a long time even with one subcutaneous, intramuscular injection, which is also directly related to life safety of people.

In addition, many studies have been carried out on a method of reducing the administration amount of an anesthetic and prolonging the duration of pain relief as much as possible in the field of anesthesia. For example, CN1382443A discloses a method of using tetrodotoxin as an anesthetic for a surgical operation, and the duration of pain relief of the anesthetic reaches about 6 hours. However, since tetrodotoxin is a kind of poisonous material and the toxicity amounts to about 1000 times of sodium cyanide, there is a problem in terms of safety, and the duration of pain relief is not sufficiently long. Besides, CN1075081A discloses a complex colloid anesthetic for external use, and the anesthetic contains chlorhexidine hydrochloride, tetracaine hydrochloride, etamsylate, carboxymethyl cellulose, liquid paraffin and gentamycin sulfate. This medicine prolonged the duration of anesthesia from about 115 minutes of the existing cases to 156 minutes, namely 2.5 hours, using colloidization of an anesthetic. In addition, many studies have been carried out to relieve pain after surgery. For example, Liu Sho Hung (China Chongging City Qianjjang industrial development zone center Hospital Anesthesiology) suggested a method for relieving pain after surgery by epidural administration. According to this method, as the result of injecting 0.5% bupivacine 5ml and dexamethasone 10mg by epidural administration after surgery, the duration of pain relief after surgery continued about 115 hours. However, for a relatively large surgery, particularly orthopedic surgery and large-scale liposuction surgery, about 10 hours after surgery is inadequate for overcoming pain, Since anesthesia just overcomes the pain of a patient at the time of surgery, an analgesic should be additionally administered during a long pain period (3∼15 days). For example, morphine, pethidine and the like should be administered, but in this case, there may be toxicity and side-effect to a patient.

In the present plastic surgery, a surgical risk due to general anesthesia, intravenous anesthesia, and lumbar anesthesia occupies 60∼70% or more of total death. Currently, in China, only first level cities are provided with anesthetic specialists and expensive anesthetic equipment, and second level, third level cities generally lack in anesthetic specialists. Thus, it is difficult to respond to on-site rescue request such as emergency and natural disaster or regional hospitals lacking traffic facilities. Thus, there is an urgent need for research and development of an analgesic for proloning the duration of action of a local anesthetic that may be directly handled and used by a doctor in charge of surgery and a medical team, and when injected in combination with a local anesthetic (many kinds of anesthetics may be used without limitation to one kind), may substantially prolong the duration of local anesthetic pain relief without injuring central nerve of the brain.

The inventors sought methods to prolong the duration of pain relief of a local anesthetic through clinical applications from 1997, filed a patent application titled "a composition for prolonging the duration of action of anesthetics" with the Chinese Patent Office on July 12, 2005 and obtained a patent on December 27, 2006 as Patent No. ZL20051008 2789.0.

In claim 1 of this patent, the composition comprises
0.1-100mg of dopamine; 1-200mg of dexamethasone; 0.5∼20mg of vitamin B complex; 10∼400mg of metronidazol; 10∼1000mg of berberine; 0.5-200mg of etamsylate; gentamicin 40,000-800,000 units; and chymotrypsin per unit of the composition.

The onset time of anethesia of the composition is 3∼5 minutes, and the duration of anethesia is 3∼9 days.

However, although the duration of anesthesia is 9 days, it does not fulfill requirements of pain relief after relatively large surgeries.

### Disclosure

### Technical Problem

The inventors repeated profound studies and experiments in order to achieve the object for further prolonging duration of pain relief of a local anesthetic, progressed selection and combination of drugs that are relatively sensitive to nerve, and progressed numerous animal experiments and clinical practices. As the result, it was proved that vitamins B, VB1, VB2, VB6, VB12, methyleneblue and dexamethasone and the like are relatively sensitive to nerve tissues and selectively act to peripheral nerve, while blocking transmission of pain sensory nerve to cause local anesthetic action. Thus, the inventors have progressed numerous studies on the way of prolonging duration of pain relief of vitamins VB, VB1, VB2, VB6, VB12, methyleneblue, dexamethasone and sodium bicarbonate.

In <<Clinical Drug Notebook>>, Jindun publisher, 1997, 9th edition, p 684, p 713, p 763, p 908 and <<Commercial Drug Notebook>> , Inmin publisher, 2007, 3rd edition, p 510 p 674, p 713, p 1089, p 1175, the properties of each drug explained above are described in detail,

VB₁ - - thiamin hydrochloride; participates in normal functions of muscle and nerve tissue.

VB₂ - - riboflavin; participates in metabolism of carbohydrate, fat and amino acid.

VB₆ - - pyridoxine hydrochloride; participates in amino acid, fat metabolism and formation of neurotransmitter.

VB₁₂ - - has the functions of improvement, nutrition supply and restoration of nerve tisse cells, and maintains integrity of nervous system myelin. It treats nervous system diseases such as neuritis, neuralgia and nerve dystrophy and the like.

Mothyleneblue - - is a redox reagent, and used for cyanide poisoning and methemoglobinemia. And, it is used for measuring urinary tract infection, urinary stone and kidney function, has local analgesic action for external use, and is used for neurodermatitis.

Dexamethasone - - has functions of preventing inflammation, alergy, virus and shock,

5% sodium bicarbonate - - is weak alkaline drug. It may neutralize and reduce stimulation of acid local pain producing substance, promotes anesthetic diffusion, and shortens onset time.

The inventors found out through large quantities of materials that dexamethasone and methyleneblue and the like further have the following actions, in addition to the actions described in the above expaliend documents. For example, dexamethasone has the action of reducing pain of local tissues, in addition to the action of preventing inflammation, allergy, shcok and virus, and it may be related to the function of reducing pressure to tissue and relieving edema. Methyleneblue is a hydrogen receptor, has relatively strong affinity to peripheral nerve after coloration, completes with other receptors to selectively act on peripheral nerve, and reversely blocks pain sensory nerve transmission to cause local analgesic action. However, in case the concentration is high and the amount is large, it may make operation view blue spot, thus making anatomy sequence indistinct and prolonging operation time, thus increasing a surgical risk. More seriously, it was proved that it may directly injure peripheral nerve medulla to cause irreparable nerve injury at high concentration (>0.03%), is harmless to human body only within normal range, and should be administered with vitamin B complex so that complementing action may be caused to effectively reduce and restore injury of nerve.

The inventors have progressed observation while optimizing drug comnbination and controlling combination ratio of drug, through animal experiments and about 7000 clinical practices, from 2006 till now. Among them, by improving the existing 8 components disclosed in the above explained patent ZL2005 1008 2789.0 to 4 components of vitamin B complex, methyleneblue, dexamethasone and sodium bicarbonate, combining the improved components with one or several local anesthetic selected from lidocaine, nupivacaine, procaine, tetracaine and prilocaine, and then subcutaneously injecting it, the effect of prolonging duration of action of a common anesthetic by the above explained 4-component composition was observed. First, the inventors administered it in one's own body and progressed comparative experiments several times to achieve effects, and then, administered it in the bodies of relatives, friends and volunteers to progress comparative experiments. After success rate reached 100% in about 30 experiment cases, it was clinically applied. Altough about 13000 surgery cases were completed from 1997 till now, no failure or toxicity and side effect cases occurred.

As the result of numerous studies and experiments, it was discovered that if vitamin B complex, methyleneblue, dexamethasone and sodium bicarbonate are combined in a specific ratio, the duration of anesthetic action may be prolonged 30∼40 days, or even longer, and the present invention is based on the discovery.

The solution means to accomplish the present invention are as follows.
1. An analgesic composition for prolonging the duration of action of a local anesthetic consisting of a vitamin B complex, methyleneblue, dexamethasone, 5% sodium bicarbonate, adrenaline hydrochloride and a 0.9% salin solution,
   wherein the components are provided in the following amounts per unit of the composition: vitamin B complex 0.5-20 mg; methyleneblue 10-20 mg; dexamethasone 10-100 mg; 5% sodium bicarbonate 2-100 ml; adrenaline hydrochloride 0.05-0.2 mg and the remaining amount of a 0.9% saline solution,
   wherein the vitamin B complex is a mixture of Vitamin B₁, Vitamin B₂, Vitamin B₆ and Vitamin B₁₂ provided in any weight proportion.
2. The composition according to the solution 1, wherein the weight proportion of Vitamin B₁ to Vitamin B₂ to Vitamin B₆ to Vitamin B₁₂ is 10-30: 1-4:10-30:10-20.

### Technical Solution

Hereinafter, the present invention will be explained in detail.

The analgesic composition for prolonging the duration of action of a local anesthetic of the present invention is a composition used in combination with an anesthetic for a surgical operation, and although the composition itself does not contain an anesthetic component, it reinforces pain relieving effect of a local anesthetic combined with the composition, shortens onset time of anesthesia, and remarkably prolongs the duration of anesthetic action.

The kinds of local anesthetics used in combination with the analgesic composition for prolonging the duration of action of local anesthetics of the present invention are not specifically limited, and for example, all common local anesthetics such as ridocain, bupivacaine, procaine, tetracaine and prilocaine may be used. Furthermore, a doctor or an anesthetic specialist may select local anesthetics according to conditions, and the local anesthetic may consist singularly or multiply.

The "0.9% saline solution" in the solution 1 itself does not have anesthetic or pain-relieving action, and is used only to control the concentration of drug when injected. And, the "remaining amount" means the amount of the saline solution needed to fill required administration amount when injected.

And, a doctor may administer 1% adrenaline hydrochloride to increase the safety of surgery according to the disease condition of a patient.

And, the use amount of the analgesic for prolonging the duration of action of a local anesthetics of the present invention may vary according to the administration amont of an anesthetic, the size of surgical site and the operation time.

The vitamins B may consist of various vitamins B in any weight proportion. In general, although vitamin B purchased from the market may be used, it is preferable that VB1, VB2, VB6 and VB12 are provided in any weight proportion. And, it is preferable that in the vitamin B, VB1, VB2, VB6 and VB12 are provided in the weight proportion of 10∼30 : 1∼4 : 10∼30 : 10∼20.

The range of the administration amount of each component refers to the amount combined with an anesthetic when anesthesia for surgical operation is conducted once. In case relative small surgery is conducted or the duration of pain is relatively short, the use amount of the analgesic composition for prolonging the duration of action of a local anesthetic may decrease as the administration amount of anesthetic decreases, and to the contrary, in case relatively big surgery is conducted or the duration of pain is relatively long, the use amount of the analgesic composition for prolonging the duration of action of a local anesthetic should be appropriately increased as the administration amount of an anesthetic increases, but all the changes are within the scope of the invention.

Although each component of the present invention is indicated as miligram, it shows the proportion instead of absolute amount. For example, even if milligrams of one or multiple components do not fall within the above range, if the proportion of component contents is appropriate, it is within the scope of the invention.

### Advantageous Effects

Compared to the existing technology in this field, the present invention has the following advantageous effects.
1. The onset time of anesthesia is rapid and takes only 60 seconds, and the duration of action of a local anesthetic continues 30∼40 days, or even longer, and thus, there is no need to additionally administer an analgesic after surgery, thus increasing safety of surgery (The onset time of a single local anesthetic requires 3∼5 minutes, and the duration of action of the anesthetic is about 60∼120 minutes).
2. When local anesthetic operation is conducted, consciousness of a patient is clear, central nerve is not injured, the influence on the main functions of the whole body is insignificant, side effects are little, and the disease conditions of a patient may be easily observed and grasped during surgery. Since nursing and transfer of a patient after surgery are easy, manpower and material power may be reduced, and it is advantageous for strengthening of function and restoration of body strength of a patient after surgery. When modern war, natural disaster, outbreak incident occur, it may be applied for on-site rescue operations (including self rescue and rescue of other people).
3. The administraton amount of a local anesthetic may be reduced, toxicity and side effects may be lowered, and safety of surgery and drug use may be increased.
4. It is used with local subcutaneous, intramuscular injection, easy to use, may be easily well-aquainted with, and thus, may be widely used in regional hospitals.
5. The application range is wide, and it may be applied for the fields of surgery, otorhinolaryngology, obsetetrics and gynegolocy, plastic surgery and local surgery of some animals. And, it is applied for pain clinic and diagnosis and treatment of cancer pain.

Hereinafter, the present invention will be explained with reference to Comparative Examples and Examples.

### Comparative Example 1: Subbrow excision

The component ratio of the analgesic composition for prolonging the duration of action of a local anesthetic is as follows.

Vitamin B complex 0.5 mg (the weight proportion of VB1, VB2, VB6 and VB12 is 10 : 2 : 10 : 10 in Vitamin B complex) and dexamethasone 10 mg.

The above components are injected into 4 ml of 0.375% bupivacaine, and a 0.9% saline solution is added so that the total volume became 8 ml, and then, the composition is subcutaneously injected into both eyebrow regions. The 8 ml local anesthetic is injected along the direction of both eyebrows by subcutaneous injection to a depth of 0.2 cm at every needle insertion point in each eyebrow region, and as the result, it was concluded that the onset time of anesthesia is 5 minutes, there is injection pain, and the duration of anesthetic action is prolonged from the existing 5∼10 hours to 72 hours.

As the results of 9 surgical procedures by this method, it was concluded that in case only vitamins B and dexamethasone are used without methyleneblue and sodium bicarbonate, ideal effect can not be acheived.

### Comparative Example 2: Augmentation mammaplsty

The component ratio of the analgesic composition for prolonging the duration of action of a local anesthetic is as follow:
Methyleneblue 20 mg and dexamethasone 30 mg.

The above components are injected into 4 ml of 0.375% bupivacaine, and a 0.9% saline solution is added so that the total volume became 8 ml, and then, the composition is injected into subcutaneous and gland tissue layer while divided into both sides along the direction of breast surgery scars, and as the result, it was concluced that the onset time of anesthesia is 5 minutes, there is injection pain, and the duration of anesthetic action is prolonged from the existing 5∼10 times to 76 hours.

As the restuls of 12 surgical procedures by this method, it was concluded that in case only methyleneblue and dexamethasone are used without vitamin B complex and sodium bicarbonate, ideal effect can not be acheived.

### Example 1: Abdominal lipoma extraction

The component ratio of the analgesic composition for prolonging the duration of action of a local anesthetic is as follows.

Vitamin B complex 10mg (the weight proportion of VB1, VB2, VB6 and VB12 is 20 : 2 : 20 : 10); methyleneblue 20 mg; 5% sodium bicarbonate 5 ml; and dexamethasone 10 mg.

The above components are injected into 15 ml of 0.375% bupivacaine, and a 0.9% saline solution is added so that the total volume became 30 ml, and then, the composition is sequentially injected into the skin and subcutaneous tissues around tumor, and as the result, it was concluded that the onset time of anesthesia is 60 seconds, there is no injection pain, and the duration of anesthetic action is prolonged from the exsiting 72 hours to 40 days (since the amount of required bupivacine may be reduced by 1/5 of the existing amount, safety may be increased).

### Example 2: Inguinal hernia repair

The component ratio of the analgesic composition for prolonging the duration of action of a local anesthetic is as follows.

Vitamin B complex 15 mg (the weight proportion of VB1, VB2, VB6 and VB12 is 20 : 2 : 20 : 15); methyleneblue 20 mg; sodium bicarbonate 5 ml; and dexamethasone 20 mg.

The above components were injected into 20 ml of 0.375% bupivacaine, and a 0.9% saline solution was added so that the total volume became 40 ml, and then, the composition was sequentially injected into the skin and subcutaneous tissues along the surgical scar line using a 3cm needle, and as the result, it was concluded that the onset time of anesthesia is 60 seconds, there is no injection pain, and the duration of anesthetic action is prolonged from the existing 72 hours to 40 days (since the amount of required bupivacine may be reduced by 1/5 of the existing amount, safety may be increased).

### Example 3: Double eyelid operation

The component ratio of the analgesic composition for prolonging the duration of action of a local anesthetic is as follows.

Vitamins B 0.5 mg (the weight proportion of VB1, VB2, VB6 and VB12 is 10 : 2 : 10 : 10); methyleneblue 10mg; dexamethasone 10mg; and 5% sodium bicarbonate 2 ml.

The above components were injected into 3ml of 1% prilocain hydrochloride according to the ratio, 0.1 mg of 1% adrenaline hydrochloride was added, and a 0.9% saline solution was added so that the total volume became 6ml, and then, the composition was injected into the eyelid region of a patient using a 2.5 cm needle, and as the result, it was concluded that the onset time of anesthesia is 60 seconds, the duration of anesthetic action continues for 40 days, and there is no injection pain (since the amount of required lidocaine is reduced by 5/1 of the existing amount, safety may be increased).

### Example 4: Mandibular angle resection

The component ratio of the analgesic composition for prolonging the duration of action of a local anesthetic is as follows.

Vitamin B complex 20 mg (the weigh ratio of VB1, VB2, VB6 and VB12 is 20 : 2 : 20 : 17); methyleneblue 20 mg; dexamethasone 30 mg; and 5% sodium bicarbonate 20 ml.

The above components were injected into 30ml of 1% prilocain hydrochloride according to the ratio, and 0.9% water for injection was added so that the total volume became 60ml, and then, the composition was subcutaneously, intramuscularly and subperiosteally injected into the operation site of a patient using a 3 cm needle, and as the result, it was concluded that onset time of anesthesia is shortened to 60 seconds from 5 minutes of the existing case wherein only lidocaine was injected, and the duration of anesthetic action is prolonged from the existing 2 hours to 40 days. Thus, it can be seen that after using the composition of the present invention, the onset time of anesthetic is remarkably shortened and the duration of anesthetic action is prolonged up to about 400 times (since the amount of required lidocaine is reduced by 5/1 of the existing amount, safety may be increased).

### Example 5: Tonsilectomy

The component ratio of the analgesic composition for prolonging the duration of action of a local anesthetic is as follows.

Vitamin B complex 0.5 mg (the weight proportion of VB1, VB2, VB6 and VB12 is 10 : 2 : 10 : 10); methyleneblue 10mg; dexamethasone 10 mg; and 5% sodium bicarbonate 2 ml.

The above components were injected into 5 ml of 0.2% tetracaine, 0.1 mg of 1% adrenaline hydrochloride was added, and a 0.9% saline solution was added so that the total volume became 10ml, and then, the composition was used for surface spray anesthesia and infiltration anesthesia around the tonsil in the oral cavity of a patient with an anesthetic gun, and as the result, it was concluded that the onset time of anethesia is 60 seconds, and the duration of pain relief after surgery is prolonged from the existing 2∼3 hours to 40 days (since the administration amount of required tetracine is decreased by 1/5, toxicity and side effects of an anesthetic may be lowered).

### Example 6: Appendectomy

The component ratio of the analgesic composition for prolonging the duration of action of local anesthetic is as follows.

Vitamin B complex 10 mg (the weight proportion of VB1, VB2, VB6 and VB12 is 20 : 2 : 20 : 15); methyleneblue 20 mg; dexamethasone 30 mg; and 5% sodium bicarbonate 5ml.

The above components were injected into 20 ml of 0.5% procaine, and a 0.9% saline solution was added so that the total volume became 40ml, and then, the composition was sequentially injected subcutaneously, intramusculary and intraperiotoneally along the appendectomy scar of the patient using a 3cm needle, and as the result, it was concluded that onset time of anesthetic is 60 seconds, and the duration of pain relief continues for 40 days. Since the patient can come down from a bed and move, complications such as intestinal adhesion may be prevented (since the amount of required procaine is reduced by 5/1 of the existing amount, safety may be increased).

### Example 7: Exploratory laparotomy

The component ratio of the analgesic composition for prolonging the duration of action of a local anesthetic is as follows.

Vitamin B complex 20 mg (the weight proportion of VB1, VB2, VB6 and VB12 is 20 : 2 : 20 : 20); methyleneblue 20 mg; dexamethasone 50 mg; and 5% sodium bicarbonate 20 ml.

The above components were injected into 50ml of 0.5% procaine according to the ratio, and a 0.9% saline solution was added so that the total volume became 100ml, and then, the composition was sequentially injected subcutaneously, intramuscularly and intraperioneally along the surgical scar in the middle of abdomen, and as the result, it was concluded that the onset time of anesthesia is 60 seconds and the duration of pain relief after surgery continues 40 days. Since a patient can come down from a bed and move, complications such as intestinal adhesion, intestinal obstruction may be prevented (by reducing the amount of required procaine by 5/1 of the existing amount, safety may be increased).

### Example 8: Face wrinkle removal surgery

The component ratio of the analgesic composition for prolonging the duration of action of a local anesthetic is as follows.

Vitamin B complex 20 mg (the weight proportion of VB1, VB2, VB6 and VB12 is 20 : 2 : 20 : 17); methyleneblue 20 mg; dexamethasone 60 mg; and 5% sodium bicarbonate 50 ml.

The above components were injected into 80 ml of 2% lidocaine according to the ratio, 0.1 mg of 1% adrenaline hydrochloride was added, and a 0.9% saline solution was added so that the total volume became 160 ml, and then, the composition was injected subcutaneously, intramuscularly and subperiosteally into the operation site of a patient using a 3 cm needle, and as the result, it was concluded that the onset time of anesthesia is shortned to 60 seconds from 5 minutes of the existing case wherein only lidocaine is injected, and the duration of pain relief is prolonged from the existing 2 hours to 40 days (by reducing the required amount of lidocaine by 1/5 of the existing amount, safety may be increased).

### Example 9: Lower body obesity liposuction

The subject was one female university student, whose height is 1.60 m and weight is 80 kg, and the component ratio of the analgesic composition for prolonging the duration of action of a local anesthetic is as follows.

Vitamin B complex 20 mg (the weight proportion of VB1, VB2, VB6 and VB12 is 20 : 2 : 20 : 20); methyleneblue 20 mg; dexamethasone 100 mg; and 5% sodium bicarbonate 100 ml.

The above components were injected into 400 ml of 2% lidocaine, and a 0.9% saline soluion was added so that the total volume became 10,000 ml, and then, the composition was subcutaneously injected into the operation site of a patient in a fan-shape using a 15∼20 cm needle, and as the result, it was concluded that the onset time of anesthesia is shortened to 60 seconds from 5 minutes of the existing case wherein only lidocaine is injected, and the duration of pain relief is prolonged from the existing 2 hours to 40 days. The surgery took 10 hours, total 8100ml fat was suctioned, the patient walked to return to a patient's room after the surgery, and the weight decreased by 15kg after 3 months.

After conducting Comparative Examples 1,2 and Examples 1∼9, the inventors further cooperated with surgeons of various fields for clinical applications in a large scale and promotions in Beijin Huangsi plastic surgery hospital, Navy General Hospital, Beijing Fu zhao medical beatuy clinic, Nanjing Dongnam University Medical Center, Shanghai Navy Hospital, Qingdao hospital, Qingdao Qingdao City Nam-gu People's hospital, Kunming plastic surgery hospital, Chongging Meilunmeihuan plastic surgery hospital, Harbin Medical University Hospital, Zhengzhou City obstetrics and hynecology/pediatrics special hospital, Xinjiang Army Medical Center, Korea Busan plastic surgery hospital and the like, and specifically, the composition of the present invention was supplied in the fields of surgery, otorhinolaryngology, oral medicine, obstetrics and hynecology, plastic surgery and pain clinic and the like. From 1997 till now, it was applied in total about 13000 casese and satisfactory effects were achieved, and no failure or toxicity and side-efect case occurred. In 2010, it was applied for local surgery of animals, and in 2010, it was also used for treatment of cancer pain, and satisfactory effects were achieved in all cases.

A surgeon may use it in combination with sinlge or complex local anesthetic according to personal circumstances when conducting surgery, and may add some additives to assure the safety and effect of surgery. For example, 1% adrenaline hydrochloride, a 0,9% salind solution for injection and the like may be added, and combining with an alkaline local anesthetic may achieve better effect. However, since such a method itself does not fall within the scope of the invention, it is not explained in detail. A surgeon may follow the prescription (combining method) of the present invention or take necessary methods and measures capable of assuring the safety of a surgical operation according to specific conditions. However, it is considered to be within the scope of the invention in case the components and proportion ranges as described in the solutions of the present invention are applied.

## Claims

1. An analgesic composition for prolonging the duration of action of a local anesthetic consisting of a vitamin B complex, methyleneblue, dexamethasone, 5% sodium bicarbonate, adrenaline hydrochloride and a 0.9% saline solution,
wherein the components are provided in the following amounts per unit of the composition: 0.5-20 mg of vitamin B complex; 10-20 mg of methyleneblue; 10-100 mg of dexamethasone; 2-100 ml of 5% sodium bicarbonate; 0.05-0.2 mg of adrenaline hydrochloride and the remaining amount of 0.9% saline solution,
wherein the vitamin B complex is a mixture of Vitamin B₁, Vitamin B₂, Vitamin B₆ and Vitamin B₁₂ provided in any weight proportion.

2. The composition according to claim 1, wherein the weight proportion of Vitamin B₁ to Vitamin B₂ to Vitamin B₆ to Vitamin B₁₂ is 10-30: 1-4:10-30:10-20.

## Patentansprüche

1. Analgetische Zusammensetzung zur Verlängerung der Wirkungsdauer eines lokalen Anästhetikums bestehend aus einem Vitamin B Komplex, Methylenblau, Dexamethason, 5 % Natriumbikarbonat, Adrenalinhydrochlorid und einer 0,9 %igen Salzlösung,
wobei die Komponenten bereitgestellt werden in den folgenden Mengen pro Einheit der Zusammensetzung: 0,5-20 mg Vitamin B Komplex; 10-20 mg Methylenblau; 10-100 mg Dexamethason; 2-100 ml von 5 % Natriumbikarbonat; 0,05-0,2 mg Adrenalinhydrochlorid und die restliche Menge 0,9 % Salzlösung,
wobei der Vitamin B Komplex eine Mischung ist aus Vitamin B₁, Vitamin B₂, Vitamin B₆ und Vitamin B₁₂, bereitgestellt in jeglichem Mengenverhältnis.

2. Zusammensetzung nach Anspruch 1, wobei das Mengenverhältnis von Vitamin B₁ zu Vitamin B₂ zu Vitamin B₆ zu Vitamin B₁₂ beträgt 10-30 : 1-4 : 10-30 : 10-20.

## Revendications

1. Composition analgésique pour prolonger la durée d'action d'un anesthésique local, constituée d'un complexe de vitamine B, de bleu de méthylène, de dexaméthasone, de bicarbonate de sodium à 5 %, de chlorhydrate d'adrénaline et d'une solution saline à 0,9 %, dans laquelle les composants sont fournis dans les quantités suivantes par unité de la composition : 0,5 à 20 mg de complexe de vitamine B ; 10 à 20 mg de bleu de méthylène ; 10 à 100 mg de dexaméthasone ; 2 à 100 ml de bicarbonate de sodium à 5 % ; 0,05 à 0,2 mg de chlorhydrate d'adrénaline et la quantité restante de solution saline à 0,9 %,
dans laquelle le complexe de vitamine B est un mélange de vitamine B₁, vitamine B₂, vitamine B₆ et vitamine B₁₂ fournies dans une proportion en poids quelconque.

2. Composition selon la revendication 1, dans laquelle la proportion en poids de vitamine B₁ à vitamine B₂ à vitamine B₆ à vitamine B₁₂ est 10-30:1-4:10-30:10-20.
